# FASCICULE DE BREVET EUROPEEN

(11) **EP 4 192 595 B1**
(45) Date de publication et mention de la délivrance du brevet: **21.02.2024**
(21) Numéro de dépôt: 21777567.5
(22) Date de dépôt: 28.09.2021
(51) Int. Cl.: B01D 3/42, B01D 3/40, B01D 3/36

(54) **PROCÉDÉ DE SÉPARATION DES MOLÉCULES D'UN MÉLANGE DE FLUIDES COMPRENANT AU MOINS UN COMPOSANT FLUORÉ**
VERFAHREN ZUR TRENNUNG VON MOLEKÜLEN AUS EINEM FLUIDGEMISCH MIT MINDESTENS EINER FLUORIERTEN KOMPONENTE
METHOD FOR SEPARATING MOLECULES FROM A FLUID MIXTURE COMPRISING AT LEAST ONE FLUORINATED COMPONENT

(30) Priorité: 12.10.2020 FR 2010422
(43) Date de publication de la demande: 14.06.2023
(73) Titulaire: Dehon, 75011 Paris (FR)
(72) Inventeur: VAN DER KELEN, Patrick, 9100 SINT-NIKLAAS (BE); DEHON, Christophe, 94360 BRY SUR MARNE (FR); SOO, Chien, Kaohsiung City 811 TAIWAN (TW)
(74) Mandataire: Argyma
(86) Numéro de dépôt international: PCT/EP2021/076716
(87) Numéro de publication internationale: WO 2022/078755

(56) Documents cités:
- WO-A1-97/03936
- WO-A2-2007/038363
- US-A- 6 047 560
- US-B2- 7 371 309

## Description

### Domaine technique

La présente invention concerne le domaine des fluides fluorés et vise plus particulièrement un procédé de séparation par distillation des molécules présentes dans un mélange de fluides comprenant au moins un fluide fluoré ainsi qu'un dispositif permettant de mettre en oeuvre le procédé.

De manière connue, un fluide fluoré est un fluide qui permet la mise en oeuvre d'un cycle thermodynamique dans un dispositif de production de froid (tel qu'une chambre froide, un surgélateur par exemple) ou dans un dispositif de production de chaud (comme une pompe à chaleur par exemple), en agissant comme propulseur d'aérosol, agent d'extinction ou agent d'expansion. Le fluide fluoré comprend communément un composant chimique pur, ou molécule pure, ou un mélange de composants chimiques.

De tels composants chimiques sont connus sous la désignation de molécules d'HydroFluoro-Carbures (HFC), composés d'atomes d'hydrogène, de fluor et de carbone, d'HydroFluoro-Oléfines (HFO) et/ou d'hydroChloroFluoro-Oléfine (HCFO). Parmi les composants HFC/HFO/HCFO, on connait principalement les R-1234yf/ze, R-134a, R-32 utilisés pour la réfrigération, la climatisation et les pompes à chaleur, les R-227ea utilisés dans les systèmes anti-incendie, les R-125, R-152a et R-143a utilisés en mélanges avec d'autres composants. Aussi, on désigne par fluide fluoré, tout fluide comprenant au moins une molécule de fluor. A ce titre, dans ce document un fluide fluoré peut comprendre ou non une molécule d'hydrocarbure.

De manière connue, les fluides fluorés en fin de vie comprennent des substances polluantes et doivent être récupérés. Par exemple, au cours d'une opération de maintenance ou de démantèlement des systèmes thermodynamiques, le dispositif est purgé de manière à récupérer le fluide fluoré usagé. Celui-ci doit ensuite être traité pour éliminer les impuretés. En outre, pour un mélange de composants chimiques, chaque composant du mélange doit être isolé afin d'être remis aux normes et permettre ainsi sa réutilisation.

De manière connue, dans un fluide fluoré usagé, les différents composants chimiques sont séparés par une méthode de distillation simple par colonnes, connue de l'homme du métier. Dans une telle distillation, un mélange initial de plusieurs composants est placé à l'état liquide dans un réservoir formant une première colonne. De manière connue, un composant est en phase vapeur au-delà de sa température d'ébullition pour une pression donnée. Etant donné que chaque fluide a une composition différente en phase gazeuse et en phase liquide, dans le cas des mélanges zéotropiques, il est possible de séparer les composants par un procédé de distillation simple.

Cependant, pour les mélanges azéotropiques ou pseudo-azéotropiques c'est-à-dire comprenant plusieurs composants présentant des compositions identiques ou quasi-identiques en phase gazeuse et en phase liquide pour une composition spécifique, une telle séparation simple n'est pas possible. Ce qui est également le cas pour les mélanges dits « pincés », c'est-à-dire les mélanges comprenant plusieurs composants présentant des compositions identiques ou presque-identiques en phase gazeuse et en phase liquide sur une zone des compositions.

Pour séparer les composants d'un mélange azéotropique il est connu deux procédés de distillation avancée soit par balance de pression (connue sous l'appellation « pressure swing » en langue anglaise), soit par extraction au moyen d'un agent entraineur, dit solvant. Dans une distillation par balance de pression, le fluide évolue successivement, en circuit continu dans deux colonnes réglées à des pressions différentes. Chaque colonne a pour rôle de séparer l'un des composants du fluide suivant la pression à laquelle elle est réglée.

Dans une distillation par extraction, un solvant est ajouté au mélange de fluides fluorés, de manière à créer une réaction physique entre le solvant et l'un des composants du fluide azéotropique ou pincé. La réaction permet alors d'isoler l'un des composants, ayant interagi avec le solvant. Le composant ayant interagi avec le solvant est alors séparé des autres composants par une simple distillation.

De nombreux documents tels que les demandes de brevets,US6047560A US7371309,

WO2007038363 ou WO9703936 décrivent la séparation de composants d'un mélange azéotropique au moyen des méthodes de distillation précitées. Cependant, chaque art antérieur cible un mélange particulier et décrit une méthode ou des solvants précis pour séparer deux ou trois composants spécifiques. Une telle approche présente un inconvénient important, parce qu'elle nécessite le traitement de chaque fluide fluoré de manière indépendante. Un tel traitement au cas par cas est chronophage et peu pratique parce qu'il nécessite l'adaptation du procédé de séparation à chaque fluide et ne permet pas un traitement global d'un mélange de plusieurs fluides fluorés comprenant des composants différents.

De plus, les molécules d'hydrofluorocarbures HFC ont un impact sur l'effet de serre. Aussi, ces dernières sont de nos jours de plus en plus remplacées par des molécules d'hydrofluoro-oléfines (HFO) ou d'hydrochlorofluoro-oléfines (HCFO), plus respectueuses de l'environnement. Concernant de telles molécules, l'art antérieur cible également à chaque fois la distillation de molécules particulières. Aussi, il n'existe pas à ce jour de méthode efficace permettant la séparation de toutes les molécules HFO ou HCFO.

L'invention vise ainsi à éliminer au moins certains de ces inconvénients en proposant un procédé de séparation des composants d'un mélange de fluides fluorés fiable et rapide permettant de séparer les composants chimiques d'un mélange azéotropique quelles que soient les molécules d'hydrofluoro-carbures ou d'hydrofluoro-oléfines qui le composent. Le procédé selon l'invention vise en outre à séparer les composants chimiques d'un mélange de plusieurs fluides fluorés de compositions différentes et pouvant contenir des hydrocarbures.

### PRESENTATION DE L'INVENTION

L'invention concerne un procédé de séparation d'une pluralité de composants chimiques d'un mélange chimique, le mélange chimique comprenant une pluralité de fluides fluorés, chaque fluide fluoré comprenant au moins un composant chimique fluoré, le procédé comprenant :
- une étape d'identification dans le mélange chimique d'au moins deux sous-mélanges, chaque sous-mélange étant un sous-mélange simple comprenant un unique composant chimique ou un sous-mélange complexe comprenant une combinaison de composants chimiques, chaque sous-mélange complexe étant associé soit à un premier groupe de distillation secondaire par une méthode de distillation par balance de pression, soit à un deuxième groupe de distillation secondaire par une méthode de distillation par extraction,
- une étape de distillation primaire, au moyen d'une colonne principale de distillation simple, de manière à séparer chaque sous-mélange identifié,
- une étape de distillation secondaire, au moyen d'une même unité avancée de deux colonnes auxiliaires d'extraction, de chaque sous-mélange complexe du premier groupe et du deuxième groupe, les sous-mélanges complexes du premier groupe étant séparés par la méthode de distillation par balance de pression et les sous-mélanges complexes du deuxième groupe étant séparés, au moyen d'un même solvant, par la méthode de distillation par extraction, par les deux mêmes colonnes auxiliaires.

Un tel procédé de séparation permet avantageusement le traitement global d'une pluralité de fluides fluorés de natures différentes et provenant de lieux différents. Aussi grâce à l'invention, il n'est plus nécessaire de traiter chaque fluide fluoré de manière indépendante, ce qui permet une séparation simple et rapide de tous les composants du mélange de fluides fluorés pouvant contenir des hydrocarbures.

De manière avantageuse, la même unité de deux colonnes auxiliaires permet à la fois de mettre en oeuvre une distillation par balance de pression et une distillation par extraction, ce qui permet de limiter le nombre de colonnes auxiliaires, ce qui représente une limitation des coûts importante.

Aussi, le procédé de séparation selon l'invention permet de séparer de manière efficace les composants chimiques d'un sous-mélange complexe quelle que soit la méthode de distillation avancée adaptée pour séparer ses composants chimiques.

Le procédé de séparation selon l'invention permet en outre avantageusement de traiter une pluralité de sous-mélanges complexes par une méthode de distillation par extraction au moyen d'un unique solvant. Aussi grâce à l'invention, il n'est pas nécessaire d'adapter le solvant aux composants chimiques présents dans chaque sous-mélange. Le solvant unique permet de séparer les composants chimiques d'un sous-mélange complexe quelles que soient les molécules chimiques qui le composent.

Grâce à l'étape d'identification préalable de chaque sous-mélange et d'association de chacun à un groupe en vue d'une distillation avancée spécifique, le procédé selon l'invention permet de classifier chaque sous-mélange avant même la première distillation. De cette façon, il est possible, grâce à l'invention, de procéder à l'étape de distillation secondaire pour les sous-mélanges complexes directement après l'étape de distillation primaire, ce qui représente un gain de temps important.

De manière préférée, chaque sous-mélange complexe est azéotropique ou pincé. Aussi, grâce à l'invention, il n'est plus nécessaire d'adapter le procédé de séparation aux composants chimiques, comme cela était le cas dans l'art antérieur.

De manière préférée, le solvant est choisi parmi les solvants suivants : le n-pentane, le dichlorométhane, la Methyl isobutyl ketone (MIBK), la Methyl ethyl ketone (MEK), la heptanone, la pentanone, la cyclohexanone et le dimethylether. De préférence, le composé utilisé est la Methyl isobutyl ketone (MIBK). Un tel solvant permet avantageusement de bénéficier d'un facteur de séparation élevé et d'une capacité d'extraction élevée pour la distillation avancée et la récupération du composant chimique.

De manière préférée, au moins un des composants chimiques d'au moins un sous-mélange est un hydrofluoro-oléfine HFO, un hydrochlorofluoro-oléfine HCFO ou un hydrofluoro-carbure HFC. Aussi, grâce à l'invention il est possible de séparer des composants hydrofluoro-oléfine HFO/HCFO de manière fiable et rapide. Le procédé de séparation selon l'invention permet de séparer tous les composants chimiques du mélange chimique quelle que soit leur nature. Aussi, même les composants hydrofluoro-oléfine HFO/HCFO peuvent être réutilisés individuellement, à la différence des méthodes de séparation de l'art antérieur qui ne permettaient pas la séparation de sous-mélanges de composants hydrofluoro-oléfine HFO/HCFO de manière efficace.

De manière préférée, chaque sous-mélange comprend un ou plusieurs composants parmi les composants chimiques suivants : R-134a, R-125, R-32, R227ea, R-143a, R22 R-1234yf, R1234ze, R1233zd.

De préférence, l'étape d'identification est réalisée à partir d'une base de données associant des sous-mélanges à une méthode de distillation secondaire par balance de pression ou à une méthode de distillation secondaire par extraction. Une telle base de données permet d'identifier de manière rapide la méthode de distillation avancée adéquate pour chaque sous-mélange de composants chimiques. En outre, après la distillation primaire, les méthodes de distillation avancées utilisées sont avantageusement des méthodes de séparation qui sont simples à mettre en oeuvre.

De manière préférée, plusieurs étapes de distillation secondaire sont réalisées successivement pour chaque sous-mélange complexe du premier groupe et du deuxième groupe.

De manière préférée, le procédé comprend une étape préliminaire de collecte d'une pluralité de fluides fluorés comprenant des composants chimiques différents et de mélange de plusieurs fluides fluorés parmi les fluides fluorés collectés, de manière à former au moins un mélange chimique de fluides fluorés. Le procédé selon l'invention permet ainsi le traitement de plusieurs mélanges chimiques résultant d'une pluralité de fluides fluorés de natures différents et collectés sur des lieux différents. Aussi, il n'est plus nécessaire de distiller chaque fluide fluoré indépendamment.

De préférence, le procédé comprend, postérieurement à l'étape préliminaire de récolte, une étape d'analyse de chaque fluide fluoré, de détection d'une pluralité de composants chimiques dans chaque fluide fluoré et de classification de la pluralité de fluides fluorés en une pluralité de mélanges prédéterminés en fonction des composants chimiques détectés.

L'invention vise également une méthode de recyclage des composants chimiques d'un mélange de fluides fluorés comprenant les étapes du procédé de séparation tel que décrit précédemment et une étape de recyclage indépendant de chaque composant chimique.

Enfin l'invention concerne un système de séparation pour la mise en oeuvre du procédé tel que décrit précédemment, le système de séparation comprenant :
- une colonne principale de distillation simple configurée pour recevoir le mélange chimique de fluides fluorés et mettre en oeuvre une distillation primaire, de manière à séparer le mélange chimique en au moins deux sous-mélanges,
- un calculateur électronique configuré pour :
   - identifier dans le mélange chimique les différents sous-mélanges, chaque sous-mélange étant un sous-mélange simple comprenant un unique composant chimique ou un sous-mélange complexe comprenant une combinaison de composants chimiques,
   - associer chaque sous-mélange complexe soit à un premier groupe de distillation secondaire par une méthode de balance de pression, soit à un deuxième groupe de distillation secondaire par une méthode de distillation par extraction,
   - une unité avancée comprenant deux colonnes auxiliaires d'extraction, configurées pour mettre en oeuvre une distillation secondaire et pour permettre à la fois une distillation par extraction au moyen d'un entraineur et une distillation par balance de pression, de manière à séparer tous les composants chimiques de chaque sous-mélange.

De préférence, le système ne comprend qu'une unique colonne principale de distillation simple et, de préférence, qu'une unique unité avancée comprenant deux colonnes auxiliaires d'extraction. Le système possède une conception minimaliste dans laquelle l'unité avancée possède plusieurs usages.

Le système selon l'invention permet avantageusement de recevoir un mélange de fluides fluorés et de séparer individuellement chaque composant chimique du mélange chimique. Le système permet en outre l'identification de plusieurs sous-mélanges avant toute distillation primaire permettant de séparer chaque sous-mélange. Le système de séparation permet la séparation de tout composant chimique même si ce dernier est inclus dans un sous-mélange azéotrope.

De manière avantageuse, le système de séparation permet au moyen de deux mêmes colonnes auxiliaires de mettre en oeuvre une distillation avancée par balance de pression ou une distillation avancée par extraction en fonction du sous-mélange que les deux colonnes auxiliaires reçoivent.

De manière préférée, l'unité avancée est constituée de deux colonnes auxiliaires, configurées pour permettre la mise en oeuvre soit d'une distillation avancée par extraction, soit d'une distillation avancée par balance de pression. Le système de séparation ne comprend ainsi que trois colonnes de distillation (une colonne principale et deux colonnes auxiliaires), ce qui représente un gain de place et une économie importante.

De manière préférée, le système de séparation comprend un réservoir de solvant configuré pour alimenter l'une des deux colonnes auxiliaires lorsque celle-ci reçoit un sous-mélange complexe du deuxième groupe.

### PRESENTATION DES FIGURES

L'invention sera mieux comprise à la lecture de la description qui va suivre, donnée uniquement à titre d'exemple, et se référant aux dessins annexés donnés à titre d'exemples non limitatifs, dans lesquels des références identiques sont données à des objets semblables et sur lesquels :
[Fig.1] La [Fig.1] est une représentation schématique d'une étape préliminaire de collecte de plusieurs fluides fluorés présentant des compositions différentes et de répartition de chaque fluide fluoré collecté en plusieurs mélanges.
[Fig.2] La [Fig.2] est une représentation schématique du système de séparation selon une forme de réalisation de l'invention.
[Fig.3] La [Fig.3] est une représentation schématique des étapes d'un procédé de séparation selon l'invention.

Il faut noter que les figures exposent l'invention de manière détaillée pour mettre en oeuvre l'invention, lesdites figures pouvant bien entendu servir à mieux définir l'invention le cas échéant.

### DESCRIPTION DETAILLEE DE L'INVENTION

L'invention concerne un procédé de séparation d'une pluralité de composants chimiques C1, C21, C22, C3, C41, C42, C51, C52 d'un mélange chimique M1-M4. Le mélange chimique M1-M4 comprend une pluralité de fluides fluorés F1-F6. Dans cet exemple, chaque fluide fluoré F1-F6 comprend au moins un composant chimique fluoré et peut comprendre ou non un ou plusieurs hydrocarbures. Par la suite par souci de concision, l'ensemble des composants chimiques C1, C21, C22, C3, C41, C42, C51, C52 seront désignés par la notation Cx.

De préférence, les différents fluides fluorés F1-F6 peuvent être de nature similaire ou différente et ont été préalablement utilisés pour permettre la mise en oeuvre d'un cycle thermodynamique dans une pluralité de dispositifs thermodynamiques indépendants. On entend par dispositif thermodynamique, un dispositif de production de froid (tel qu'une chambre froide, un surgélateur ou un réfrigérateur), un dispositif de production de chaud (comme une pompe à chaleur), ou bien encore un propulseur d'aérosol par exemple.

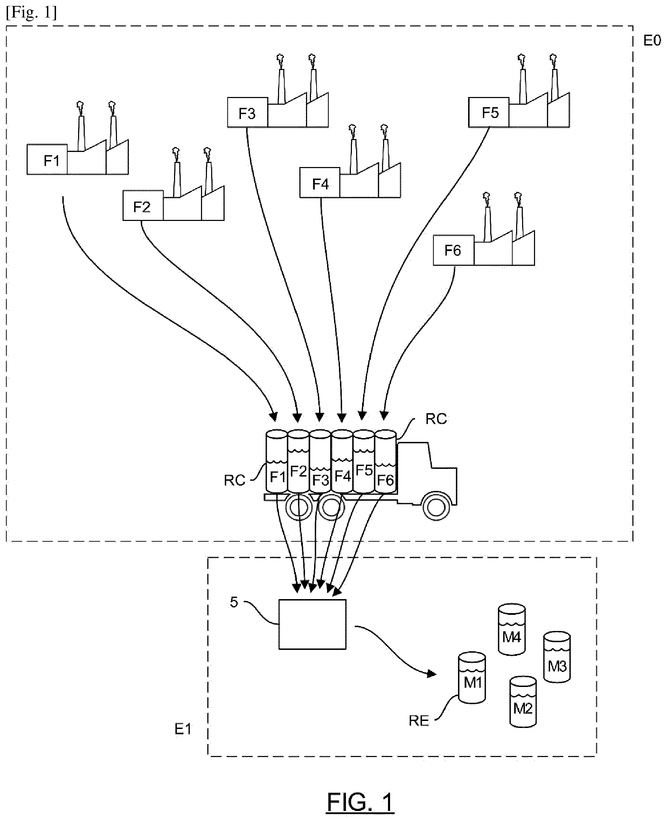

En référence à la [Fig.1], tous les fluides fluorés F1-F6 ont préalablement été récoltés dans des réservoirs de collecte RC distincts au cours d'une opération de récupération collective, comme cela sera décrit plus en détails par la suite.

Ce document présente l'exemple de six fluides fluorés F1-F6, cependant il va de soi que le nombre de fluides fluorés F1-F6 récoltés peut être différent en particulier supérieur à six.

Chaque fluide fluoré F1-F6 comprend un composant chimique Cx pur ou un mélange de composants chimiques Cx.

De préférence, chaque composant chimique Cx est une molécule chimique de type hydrofluoro-carbure HFC, hydrochlorofluoro-carbure HCFC, hydrofluoro-oléfine HFO ou hydrochlorofluoro-oléfine HCFO. Par souci de concision, dans la suite de ce document, les composants hydrofluoro-oléfine, désignées HFO/HCFO, se réfèrent tout aussi bien aux hyrdofluoro-oléfine HFO qu'aux hydrochlorofluoro-oléfine HCFO. De préférence encore, au moins un des mélanges chimiques M1-M4 comprend au moins une molécule d'hydrofluoro-oléfine HFO/HCFO.

Dans cet exemple, les composants HFC et HCFC sont de préférence choisis parmi la liste de composants suivants : R-134a, R-125, R-32, R227ea, R-143a et R22. De même dans cet exemple, les composants HFO/HCFO sont de préférence choisis parmi la liste de composants suivants : R-1234yf, R1234ze, R1233zd.

Dans cet exemple, toujours en référence à la [Fig.1], une fois récolté, chaque fluide fluoré F1-F6 est configuré pour être analysé par un système de triage, se présentant sous la forme d'un dispositif électronique 5 et comportant des capteurs de différents types pour classer les fluides fluorés F1-F6 en fonction de ses propriétés et de sa nature.

Dans cet exemple, tous les fluides fluorés F1-F6 sont classés en quatre groupes, correspondant chacun à une liste de composants parmi les composants cités précédemment. L'ensemble des fluides fluorés F1-F6 d'un même groupe sont configurés pour être mélangés, de manière à former un ou plusieurs mélanges de fluides fluorés F1-F6, dans cet exemple quatre mélanges chimiques M1-M4.

Dans cet exemple, les quatre mélanges chimiques M1-M4 comprennent de préférence les composants répertoriés dans la table 1 suivante, parmi les composants chimiques ciblés par le système de séparation S selon l'invention (il va de soi que chaque fluide fluoré F1-F6 peut comprendre certains composants chimiques non ciblés par l'invention, comme par exemple les R12, R152a, R290, R600, R600a, R601a, RE170) :

**[Tableaux1]**

| Mélange | Composants autorisés | Composants autorisés en quantité limitée, c'est-à-dire de préférence inférieure à 20% | Composants non autorisés |
|---|---|---|---|
| M1 | R32, R125, R134a, R1234yf, R1234ze | / | R143a, R227ea, R22 |
| M2 | R125, R143a, R134a | R1234ze, R227ea | R32, R1234yf, R22 |
| M3 | R22 | R32, R125, R143a, R134a, R1234yf, R1234ze, R227ea | / |
| M4 | R227ea | R32, R125, R143a, R134a, R22 | R1234yf, R1234ze |

Dans cet exemple, chaque mélange chimique M1-M4 résulte ainsi d'un unique fluide fluoré F1-F6 récolté ou du mélange de plusieurs fluides fluorés F1-F6 récoltés. Chaque mélange chimique M1-M4 sera traité de préférence de manière indépendante par le système de séparation S selon l'invention. Dans l'art antérieur, chaque fluide fluoré F1-F6 devait être traité de manière totalement indépendante, et la récupération et la séparation des composants étaient individuelles et non collectives.

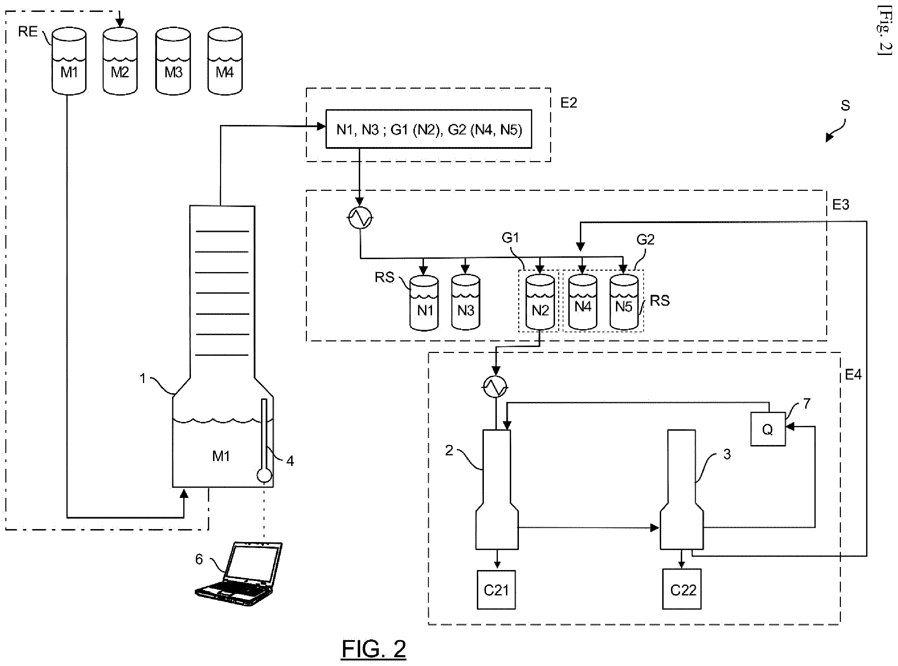

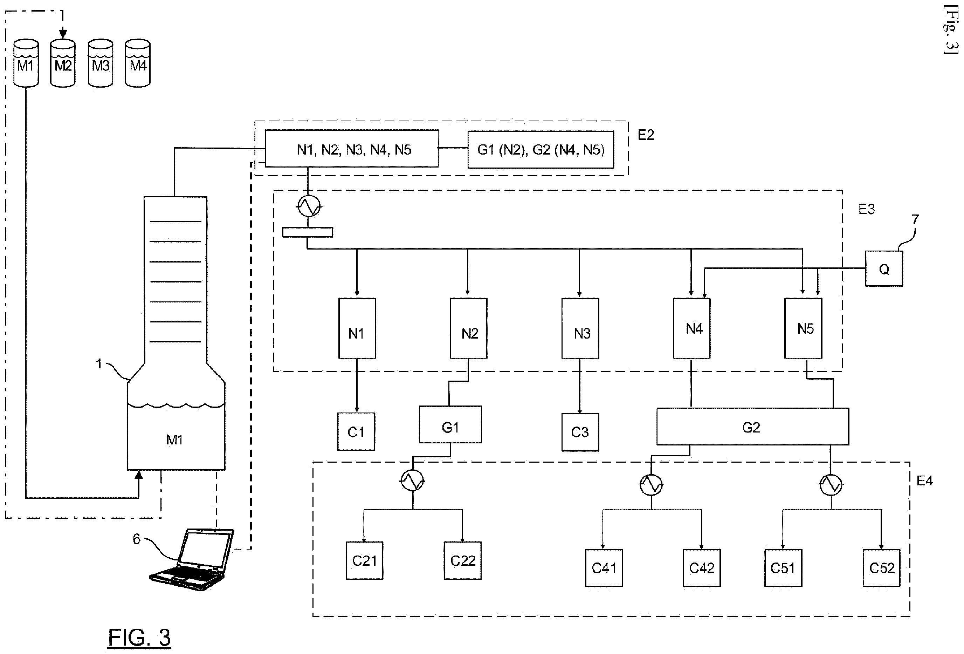

Selon l'invention, en référence aux figures 2 et 3, chaque mélange chimique M1-M4 est configuré pour être séparé dans une distillation primaire en une pluralité de sous-mélanges N1-N5, chaque sous-mélange N1-N5 comprend soit un composant chimique Cx pur, soit une combinaison de composants chimiques Cx. Une telle opération sera décrite plus en détails dans la suite de ce document.

Dans cet exemple, comme représenté sur la [Fig.3], le mélange chimique M1 comprend cinq sous-mélanges N1-N5, chaque sous-mélange N1-N5 comprend les composants chimiques Cx ou les combinaisons de composants chimiques Cx suivants :
- le sous-mélange N1 est un sous-mélange simple comprenant un unique composant chimique C1 pur ;
- le sous-mélange N2 est un sous-mélange complexe comprenant une composition azéotrope de deux composants chimiques C21, C22 ;
- le sous-mélange N3 est un sous-mélange simple comprenant un unique composant chimique C3 pur ;
- le sous-mélange N4 est un sous-mélange complexe comprenant une composition azéotrope de deux composants chimiques C41, C42 ; et
- le sous-mélange N5 est un sous-mélange complexe comprenant une composition azéotrope de deux composants chimiques C51, C52.

Par souci de simplicité et de concision, chaque sous-mélange complexe N2, N4, N5 comprend dans ce document deux composants chimiques C21, C22, C41, C42, C51, C52. Cependant, il va de soi que chaque sous-mélange complexe peut comprendre un nombre différent de composants chimiques, en particulier trois composants chimiques.

Dans une forme de réalisation préférée, chaque sous-mélange N2, N4, N5 comprenant une combinaison de composants chimiques Cx est azéotrope ou pincé.

Le procédé de séparation selon l'invention est réalisé au moyen d'un système de séparation S selon l'invention.

En référence à la [Fig.2], le système de séparation S selon l'invention comprend une colonne principale 1 de récupération d'un mélange chimique M1-M4 de fluides fluorés F1-F6, configurée pour mettre en oeuvre une distillation simple (appelée également distillation primaire), et une unité avancée de deux colonnes auxiliaires 2, 3 d'extraction, configurées pour mettre en oeuvre une distillation avancée (appelée également distillation secondaire). Dans le système de séparation selon l'invention, les deux mêmes colonnes auxiliaires 2, 3 de l'unité avancée permettent à la fois une distillation extractive au moyen d'un entraineur et une distillation par balance de pression. L'unité avancée comprend ainsi une première colonne auxiliaire 2 et une deuxième colonne auxiliaire 3.

La colonne principale 1 est configurée pour recevoir l'un des mélanges chimiques M1-M4 de fluides fluorés F1-F6 préalablement classés. La colonne principale 1 est reliée à l'aide de tuyauteries appropriées à une pluralité de réservoirs de stockage RS, configuré chacun pour recevoir l'un des sous-mélanges N1-N5 du mélange chimique M1-M4, après l'opération de distillation simple.

La colonne principale 1 permet directement l'extraction de chaque composant pur C1, C3, après la distillation simple et la séparation des sous-mélanges N1-N5, notamment des sous-mélanges simples N1, N3, comme cela est représenté sur la [Fig.3].

En référence à la [Fig.2], la première colonne auxiliaire 2 de l'unité avancée est reliée à l'aide de tuyauteries appropriées à l'un des réservoirs de stockage RS de l'un des sous-mélanges N2, N4, N5 complexes et est configurée pour recevoir successivement chaque sous-mélange N2, N4, N5, comme cela sera également décrit plus en détails par la suite.

La deuxième colonne auxiliaire 3 de l'unité avancée est reliée directement à la première colonne auxiliaire 2, comprenant l'un des sous-mélanges N2, N4, N5 complexes comprenant une combinaison de composants chimiques C21, C22, C41, C42, C51, C52.

Le système de séparation S comprend en outre un dispositif d'identification 4 de la pluralité de sous-mélanges N1-N5 à partir de l'ensemble des composants chimiques Cx présents dans le mélange chimique M1-M4. Le dispositif d'identification 4 se présente par exemple sous la forme d'un système d'analyse qualitative et quantitative (par exemple un système d'analyse chromatographique) des mélanges M1-M4 et des sous-mélanges N2, N4, N5. Ainsi, le dispositif d'identification 4 est configuré pour détecter chaque composant chimique Cx présent dans le mélange chimique M1-M4 et dans chaque sous-mélange N2-N4-N5.

Selon une forme de réalisation de l'invention, le système de séparation S comprend également un calculateur électronique 6 extérieur, par exemple un ordinateur, relié électriquement au dispositif d'identification 4. Le calculateur électronique 6 est configuré pour déterminer, pour chaque sous-mélange N1-N5 identifié, s'il s'agit d'un sous-mélange N1-N5 simple comprenant un unique composant chimique Cx pur ou un sous-mélange N1-N5 complexe comprenant une combinaison de composants chimiques Cx. Le calculateur électronique 6 est en outre configuré pour déterminer pour chaque sous-mélange N1-N5 complexe, une méthode de distillation avancée pour permettre la séparation des composants chimiques Cx de la composition azéotrope de chacun. La méthode de distillation avancée est déterminée selon l'invention parmi une méthode de distillation par balance de pression et une méthode de distillation par extraction au moyen d'un solvant. Ces méthodes de distillation sont connues de l'homme du métier et ne seront pas décrites plus en détails dans ce document. Autrement dit, le calculateur électronique 6 permet de coordonner le traitement successif des sous-mélanges N1-N5 afin de séparer les composants Cx.

De préférence, pour déterminer la méthode de distillation avancée adéquate pour chaque sous-mélange N1-N5 complexe, le calculateur électronique 6 est configuré, pour identifier dans une base de données (voir table 3), chacune des combinaisons de composants chimiques ainsi que la méthode de distillation avancée associée à chacune. Autrement dit, une telle base de données comprend l'ensemble des composants chimiques Cx purs et des combinaisons de composants chimiques Cx potentiellement présents dans un fluide fluoré F1-F6, ainsi que la méthode de distillation avancée qui permet de séparer chaque combinaison de composants chimiques Cx. Le calculateur électronique 6 est en outre configuré pour classifier chaque sous-mélange N1-N5 complexe en associant chacun à un premier groupe G1 comprenant les sous-mélanges N1-N5 (dans cet exemple uniquement le sous-mélange N2) qui doivent être distillés par la méthode avancée par balance de pression ou à un deuxième groupe G2 comprenant les sous-mélanges N1-N5 (dans cet exemple les sous-mélanges N4 et N5) qui doivent être distillés par la méthode avancée par extraction.

Le système de séparation S comporte en outre un réservoir de solvant 7 configuré pour alimenter la première colonne auxiliaire 2 lorsque celle-ci est destinée à subir une méthode de distillation par extraction au moyen d'un solvant. Le réservoir de solvant 7 comporte un solvant Q (également désigné entraineur) choisi parmi les cétones, les alcanes halogénés (C4 à C7), les alcanes (C4 à C7) et les éthers. De préférence, le solvant Q est choisi parmi la liste suivante : le n-pentane, le dichlorométhane, la Methyl isobutyl ketone (MIBK), la Methyl ethyl ketone (MEK), la heptanone, la pentanone, la cyclohexanone et le dimethylether. De préférence encore, le solvant Q utilisé est le Methyl isobutyl ketone étant donné qu'il présente des avantages au niveau de la puissance de sélectivité et de la récupération.

Il va dorénavant être décrit un procédé de séparation des composants chimiques Cx présents dans le mélange chimique M1-M4 de fluides fluorés F1-F6 selon l'invention, en référence aux figures 1 à 3. Le procédé de séparation selon l'invention vise à séparer individuellement la pluralité de composants chimiques Cx présents dans un mélange chimique M1-M4 tels que décrits précédemment. Chaque mélange M1-M4 est introduit successivement dans le système de séparation S.

De préférence, le procédé de séparation selon l'invention, permet la séparation de tous les composants chimiques Cx d'un mélange chimique M1-M4 dans lequel chaque composant chimique Cx peut être soit un hydrofluorocarbure HFC, soit un hydrochlo-rofluorocarbure HCFC, soit un hydrofluoro-oléfine HFO/HCFO.

En référence à la [Fig.1], le procédé de séparation comprend une étape préliminaire de collecte E0 d'une pluralité de fluides fluorés F1-F6. Dans cette étape, un opérateur se déplace par exemple sur différents lieux pour récolter les fluides fluorés F1-F6 d'une pluralité de dispositifs thermodynamiques différents. A titre d'exemple, chaque fluide fluoré F1-F6 est récolté au cours d'une opération de maintenance de systèmes de climatisation, dans l'agriculture ou lors de purges d'aérosols par exemple.

Dans cette étape préliminaire E0, l'opérateur récupère chaque fluide fluoré F1-F6 dans une pluralité de réservoirs de collecte RC différents.

Chaque fluide fluoré F1-F6 est alors analysé dans une étape E1, par un dispositif électronique 5, afin de déterminer quels composants chimiques Cx sont présents dans chaque fluide fluoré F1-F6. Dans cette première étape E1, les fluides fluorés F1-F6 sont classés, dans cet exemple, en quatre groupes puis mélangés, de manière à former quatre mélanges chimiques M1-M4. Chaque mélange chimique M1-M4 représente un ensemble de composants chimiques Cx prédéterminés. Suivant les composants chimiques Cx présents dans l'ensemble des fluides fluorés F1-F6, il va de soi que chaque mélange chimique M1-M4 peut comprendre un mélange de plusieurs fluides fluorés F1-F6, un seul fluide fluoré F1-F6 ou bien aucun fluide fluoré F1-F6. Les mélanges chimiques M1-M4 permettent avantageusement un traitement global simultané de plusieurs fluides fluorés F1-F6 et non un traitement au cas par cas.

Tous les mélanges chimiques M1-M4 sont alors stockés dans des récipients RE indépendants. Chaque mélange chimique M1-M4 sera traité indépendamment. Par la suite, le procédé de séparation sera décrit pour la séparation des composants chimiques Cx présents dans le mélange chimique M1.

En référence à la [Fig.3], le mélange chimique M1 est transféré dans la colonne principale 1 du système de séparation S.

La composition du mélange chimique M1 est analysée via le dispositif d'identification 4 relié au calculateur électronique 6. Le procédé comprend alors une étape d'identification E2 dans le mélange chimique M1 d'une pluralité de sous-mélanges N1-N5 par le calculateur électronique 6. Chaque sous-mélange N1-N5 comprend un unique composant chimique Cx ou une combinaison de composants chimiques Cx. Comme décrit précédemment, dans cet exemple représenté sur la [Fig.3], cinq sous-mélanges N1-N5 sont identifiés et comprennent les composants répertoriés dans la table 2 suivante.

**[Tableaux2]**

| Mélange | Sous-mélanges identifiés | Composant pur ou combinaison de composants |
|---|---|---|
| | | |
| M1 | N1 | C1 |
| | N2 | C21, C22 |
| | N3 | C3 |
| | N4 | C41, C42 |
| | N5 | C51, C52 |

La détermination des sous-mélanges N1-N5 est avantageuse étant donné qu'elle permet de prédéterminer si le sous-mélange N1-N5 nécessite une distillation avancée et quelle méthode de distillation avancée sera nécessaire pour séparer tous les composants chimiques.

Le nombre et la composition des sous-mélanges N1-N5 ne sont donnés ici qu'à titre d'exemple, cependant il va de soi que le nombre de sous-mélanges N1-N5 dans chaque mélange chimique M1-M4 peut être différent.

Les sous-mélanges N1-N5 complexes identifiés sont également classifiés de manière à être séparés en un premier groupe G1 et un deuxième groupe G2. Selon l'invention, le premier groupe G1 comprend l'ensemble des sous-mélanges (dans cet exemple uniquement le sous-mélange N2) comprenant une combinaison de composants chimiques Cx qui peuvent être séparés par une méthode de distillation avancée par balance de pression. Le deuxième groupe G2 comprend l'ensemble de sous-mélanges (dans cet exemple les sous-mélanges N4, N5) comprenant une combinaison de composants chimiques Cx qui peuvent être séparés par une méthode de distillation avancée par extraction.

Une telle classification est réalisée de préférence par le calculateur électronique 6, à partir de la base de données. Dans cet exemple, le calculateur électronique 6 permet d'établir les identifications et classifications répertoriées dans la table 3 suivante grâce à sa base de données :

**[Tableaux3]**

| Sous-mélanges | Composants | Groupe | Méthode de distillation |
|---|---|---|---|
| N1 | C1 | / | / |
| N2 | C21, C22 | G1 | Distillation par balance de pression |
| N3 | C3 | / | / |
| N4 | C41, C42 | G2 | Distillation par extraction |
| N5 | C51, C52 | G2 | Distillation par extraction |

Toujours en référence à la [Fig.3], le procédé de séparation comprend ensuite une étape de distillation primaire E3, par la colonne principale 1 de distillation, du mélange chimique M1, de manière à séparer chaque sous-mélange N1-N5 identifié dans un réservoir de stockage RS indépendant.

Le procédé comprend alors une étape de distillation secondaire E4 de chaque sous-mélange complexe N2, N4, N5 au moyen de l'unité avancée des deux colonnes auxiliaires 2, 3. Plus précisément, cette étape permet la distillation avancée de chaque sous-mélange N1-N5 complexe du premier groupe G1, dans cet exemple uniquement le sous-mélange N2, et de chaque sous-mélange N1-N5 complexe du deuxième groupe G2, dans cet exemple les sous-mélanges N4 et N5. Chaque sous mélange complexe N2, N4, N5 est distillé de manière indépendante, aussi tous les sous-mélanges complexes N2, N4, N5 sont soumis successivement à une distillation secondaire par l'unité avancée des deux colonnes auxiliaires 2, 3.

Dans cette étape de distillation secondaire E4, le sous-mélange complexe N2 du premier groupe G1 est distillé par la méthode par balance de pression, de manière à séparer les composants chimiques C21, C22 du sous-mélange N2 complexe du premier groupe G1.

Dans cette étape, comme cela est connu, la première colonne auxiliaire 2 est réglée à une première pression et la deuxième colonne auxiliaire 3 est réglée à une deuxième pression, strictement différente de la première pression pour permettre la séparation de différents composants C21, C22.

Dans cette étape de distillation secondaire E4, chaque sous-mélange complexe N4, N5 du deuxième groupe G2 est distillé par la méthode de distillation par extraction, au moyen du même solvant Q, de manière à séparer les composants chimiques C41, C42, C51, C52 de chaque sous-mélange complexe N4, N5 du deuxième groupe G2.

Lorsque le sous-mélange complexe N4, N5 doit être séparé par la méthode de distillation par extraction, le solvant Q est alors injecté dans la première colonne auxiliaire 2. Le solvant Q passe ensuite dans la deuxième colonne auxiliaire 3 et est ensuite récupéré dans le réservoir de solvant 7 de manière à être recyclé pour être utilisé dans une prochaine distillation par extraction. En effet, dans un mode de mise en oeuvre préféré, tous les sous-mélanges complexes N4, N5 du deuxième groupe G2 sont distillés au moyen d'un unique solvant Q. De préférence, le solvant Q est l'un des solvants suivants : le n-pentane, le dichlorométhane, la Methyl isobutyl ketone (MIBK), la Methyl ethyl ketone (MEK), la heptanone, la pentanone, la cyclohexanone et le dimethylether. De préférence encore, le solvant Q utilisé est le Methyl isobutyl ketone. De manière préférée, le réservoir de solvant 7 est relié directement à la première colonne auxiliaire 2 d'extraction, de manière à l'alimenter en solvant Q. De même, le réservoir de solvant 7 est alimenté en retour par le solvant Q, récupéré directement de la deuxième colonne auxiliaire 3 d'extraction (comme représenté sur la [Fig.2]).

Une telle méthode de distillation par extraction est avantageuse étant donné qu'un unique solvant Q est utilisé, ce qui réduit les coûts matériels et de logistique. Ainsi, tout type de mélange M1-M4 peut être traité de manière dynamique, les étapes de distillation étant réalisées en fonction des sous-mélanges N1-N5 identifiés dynamiquement.

A l'issue du procédé de séparation selon l'invention, chaque composant chimique Cx présent dans le mélange chimique M1 de fluides fluorés F1-F6 est séparé des autres composants chimiques Cx. Les autres mélanges chimiques M2-M4 sont ensuite successivement introduits dans la colonne principale 1 et les étapes E2 à E4 sont répétées afin de séparer individuellement chaque composant Cx de chaque mélange chimique M2-M4.

Un tel procédé selon l'invention permet avantageusement de séparer de manière efficace des composants chimiques d'un mélange de plusieurs fluides fluorés, sans nécessiter le traitement au cas par cas de chaque fluide fluoré qui nécessiterait une adaptation du système de séparation S à chaque fluide réfrigérant.

Grâce au procédé de séparation selon l'invention, les composants chimiques de plusieurs fluides fluorés provenant de dispositifs thermodynamiques différents peuvent être séparés au moyen d'un unique système de séparation, ce qui représente un gain de temps important.

De plus, au cours du procédé de séparation, un même système de séparation peut être utilisé successivement pour différents mélanges chimiques de fluides fluorés, ce qui permet de limiter les coûts de manière importante, parce qu'une même unité de deux colonnes d'extraction peut être utilisée quelle que soit la méthode de distillation avancée nécessaire pour séparer les différents composants du mélange chimique.

L'invention concerne également une méthode de recyclage de chaque composant chimique. Pour cela, suite aux différentes étapes du procédé, la méthode de recyclage comprend une étape de recyclage de chaque composant chimique séparé pour être réutilisé ultérieurement.

## Revendications

1. Procédé de séparation d'une pluralité de composants chimiques (Cx) d'un mélange chimique (M1-M4), le mélange chimique (M1-M4) comprenant une pluralité de fluides fluorés (F1-F6), chaque fluide fluoré (F1-F6) comprenant au moins un composant chimique (Cx) fluoré, le procédé comprenant :
• une étape d'identification (E2) dans le mélange chimique (M1-M4) d'au moins deux sous-mélanges (N1-N5), chaque sous-mélange (N1-N5) étant un sous-mélange simple (N1, N3) comprenant un unique composant chimique (C1, C4) ou un sous-mélange complexe (N2, N4, N5) comprenant une combinaison de composants chimiques (C21, C22, C41, C42, C51, C52), chaque sous-mélange complexe (N2, N4, N5) étant associé soit à un premier groupe (G1) de distillation secondaire par une méthode de distillation par balance de pression, soit à un deuxième groupe (G2) de distillation secondaire par une méthode de distillation par extraction,
• une étape de distillation primaire (E3), au moyen d'une colonne principale (1) de distillation simple, de manière à séparer chaque sous-mélange (N1-N5) identifié,
• une étape de distillation secondaire (E4), au moyen d'une même unité avancée de deux colonnes auxiliaires (2, 3) d'extraction, de chaque sous-mélange complexe (N2, N4, N5) du premier groupe (G1) et du deuxième groupe (G2), les sous-mélanges complexes (N2) du premier groupe (G1) étant séparés par la méthode de distillation par balance de pression et les sous-mélanges complexes (N4, N5) du deuxième groupe (G2) étant séparés, au moyen d'un même solvant (Q), par la méthode de distillation par extraction, par les deux mêmes colonnes auxiliaires (2, 3).

2. Procédé de séparation selon la revendication 1, dans lequel chaque sous-mélange complexe (N2, N4, N5) est azéotropique ou pincé.

3. Procédé de séparation selon l'une des revendications 1 et 2, dans lequel, le solvant (Q) est choisi parmi les solvants suivants : le n-pentane, le dichlorométhane, la Methyl isobutyl ketone (MIBK), la Methyl ethyl ketone (MEK), la heptanone, la pentanone, la cyclohexanone et le dimethylether.

4. Procédé de séparation selon l'une des revendications 1 à 3, dans lequel au moins un des composants chimiques (Cx) d'au moins un sous-mélange (N1-N5) est un hydrofluoro-oléfine HFO, un hydrochlorofluoro-oléfine HCFO, un hydrofluoro-carbure HFC ou un hydrochlorofluoro-carbure HCFC.

5. Procédé de séparation selon l'une des revendications 1 à 4, dans lequel l'étape d'identification (E2) est réalisée à partir d'une base de données associant des sous-mélanges à une méthode de distillation secondaire par balance de pression ou à une méthode de distillation secondaire par extraction.

6. Procédé de séparation selon l'une des revendications 1 à 5, comprenant une étape préliminaire de collecte (E0) d'une pluralité de fluides fluorés (F1-F6) comprenant des composants chimiques (Cx) différents et de mélange de plusieurs fluides fluorés (F1-F6) parmi les fluides fluorés (F1-F6) collectés, de manière à former au moins un mélange chimique (M1-M4) de fluides fluorés (F1-F6).

7. Méthode de recyclage des composants chimiques (Cx) d'un mélange chimique (M1-M4) de fluides fluorés (F1-F6) comprenant les étapes (E1-E4) du procédé de séparation selon l'une des revendications 1 à 6 et une étape de recyclage indépendant de chaque composant chimique (Cx).

8. Système de séparation (S) pour la mise en oeuvre du procédé selon l'une des revendications 1 à 6, le système de séparation (S) comprenant :
• une colonne principale (1) de distillation simple, configurée pour recevoir le mélange chimique (M1-M4) de fluides fluorés (F1-F6) et mettre en oeuvre une distillation primaire, de manière à séparer le mélange chimique (M1-M4) en au moins deux sous-mélanges (N1-N5),
• un calculateur électronique (6) configuré pour :
• identifier dans le mélange chimique (M1-M4) les différents sous-mélanges (N1-N5), chaque sous-mélange (N1-N5) étant un sous-mélange simple (N1, N3) comprenant un unique composant chimique (C1, C3) ou un sous-mélange complexe (N2, N4, N5) comprenant une combinaison de composants chimiques (C21, C22, C41, C42, C51, C52),
• associer chaque sous-mélange complexe (N2, N4, N5) soit à un premier groupe (G1) de distillation secondaire par une méthode de balance de pression, soit à un deuxième groupe (G2) de distillation secondaire par une méthode de distillation par extraction,
• une unité avancée comprenant deux colonnes auxiliaires (2, 3) d'extraction, configurées pour mettre en oeuvre une distillation secondaire et pour permettre à la fois une distillation par extraction au moyen d'un entraineur et une distillation par balance de pression, de manière à séparer tous les composants chimiques (C21, C22, C41, C42, C51, C52) de chaque sous-mélange (N2, N4, N5).

9. Système de séparation (S) selon la revendication 8 dans lequel l'unité avancée est constituée de deux colonnes auxiliaires (2, 3), configurées pour permettre la mise en oeuvre soit d'une distillation avancée par extraction, soit d'une distillation avancée par balance de pression.

10. Système de séparation (S) selon l'une des revendications 8 et 9 comprenant un réservoir de solvant (7) configuré pour alimenter l'une des deux colonnes auxiliaires (2, 3) lorsque celle-ci reçoit un sous-mélange (N4, N5) complexe du deuxième groupe (G2).

## Patentansprüche

1. Verfahren zur Trennung einer Vielzahl chemischer Komponenten (Cx) aus einem chemischen Gemisch (M1-M4), wobei das chemische Gemisch (M1-M4) eine Vielzahl fluorierter Fluide (F1-F6) umfasst, wobei jedes fluorierte Fluid (F1-F6) mindestens eine fluorierte chemische Komponente (Cx) umfasst, wobei das Verfahren umfasst:
• einen Identifikationsschritt (E2) in dem chemischen Gemisch (M1-M4) von mindestens zwei Untergemischen (N1-N5), wobei jedes Untergemisch (N1-N5) ein einfaches Untergemisch (N1, N3) ist, das eine einzige chemische Komponente (C1, C4) umfasst oder ein komplexes Untergemisch (N2, N4, N5), das eine Kombination chemischer Komponenten (C21, C22, C41, C42, C51, C52) umfasst, wobei jedes komplexe Untergemisch (N2, N4, N5) entweder einer ersten Gruppe sekundärer Destillation (G1) durch eine Zweidruck-Destillationsmethode oder einer zweiten Gruppe sekundärer Destillation (G2) durch eine Extraktions-Destillationsmethode zugeordnet ist,
• einen Primärdestillationsschritt (E3) mittels eine einfachen Hauptdestillationssäule (1) derart, dass jedes identifizierte Untergemisch (N1-N5) getrennt wird,
• einen Sekundärdestillationsschritt (E4) mittels einer selben fortgeschrittenen Einheit aus zwei Extraktions-Hilfssäulen (2, 3) jedes komplexen Untergemischs (N2, N4, N5) der ersten Gruppe (G1) und der zweiten Gruppe (G2), wobei die komplexen Untergemische (N2) der ersten Gruppe (G1) durch die Zweidruck-Destillationsmethode getrennt werden und die komplexen Untergemische (N4, N5) der zweiten Gruppe (G2) mittels desselben Lösungsmittels (Q) durch die Extraktions-Destillationsmethode durch die zwei gleichen Hilfssäulen (2, 3) getrennt werden.

2. Trennverfahren nach Anspruch 1, wobei jedes komplexe Untergemisch (N2, N4, N5) azeotrop oder gepincht ist.

3. Trennverfahren nach einem der Ansprüche 1 und 2, wobei das Lösungsmittel (Q) aus den folgenden Lösungsmitteln ausgewählt ist: n-Pentan, Dichlormethan, Methylisobutylketon (MIBK), Methylethylketon (MEK), Heptanon, Pentanon, Cyclohexanon und Dimethylether.

4. Trennverfahren nach einem der Ansprüche 1 bis 3, wobei mindestens eine der chemischen Komponenten (Cx) mindestens eines Untergemischs (N1-N5) ein Hydrofluorolefin HFO, ein Hydrochlorfluorolefin HCFO, ein Fluorkohlenwasserstoff FKW oder ein Fluorchlorkohlenwasserstoff FCKW ist.

5. Trennverfahren nach einem der Ansprüche 1 bis 4, wobei der Identifikationsschritt (E2) auf der Basis einer Datenbank durchgeführt wird, die Untergemische einer sekundären Zweidruck-Destillationsmethode oder einer sekundären Extraktions-Destillationsmethode zuordnet.

6. Trennverfahren nach einem der Ansprüche 1 bis 5, umfassend einen vorbereitenden Schritt des Sammelns (E0) einer Vielzahl fluorierter Fluide (F1-F6), die unterschiedliche chemische Komponenten (Cx) umfassen, und des Mischens mehrerer fluorierter Fluide (F1-F6) von den gesammelten fluorierten Fluiden (F1-F6) derart, dass mindestens ein chemisches Gemisch (M1-M4) fluorierter Fluide (F1-F6) gebildet wird.

7. Methode zum Recyceln der chemischen Komponenten (Cx) eines chemischen Gemischs (M1-M4) fluorierter Fluide (F1-F6), umfassend die Schritte (E1-E4) des Trennverfahrens nach einem der Ansprüche 1 bis 6 und einen unabhängigen Recyclingschritt jeder chemischen Komponente (Cx).

8. Trennsystem (S) für die Durchführung des Verfahrens nach einem der Ansprüche 1 bis 6, wobei das Trennsystem (S) umfasst:
• eine einfache Hauptdestillationssäule (1), die zur Aufnahme des chemischen Gemischs (M1-M4) fluorierter Fluide (F1-F6) und Durchführung einer Primärdestillation derart ausgelegt ist, dass das chemische Gemisch (M1-M4) in mindestens zwei Untergemische (N1-N5) getrennt wird,
• einen elektronischen Rechner (6), der ausgelegt ist, um:
• in dem chemischen Gemisch (M1-M4) die verschiedenen Untergemische (N1-N5) zu identifizieren, wobei jedes Untergemisch (N1-N5) ein einfaches Untergemisch (N1, N3) ist, das eine einzige chemische Komponente (C1, C3) umfasst oder ein komplexes Untergemisch (N2, N4, N5), das eine Kombination chemischer Komponenten (C21, C22, C41, C42, C51, C52) umfasst,
• jedem komplexen Untergemisch (N2, N4, N5) entweder einer ersten Gruppe sekundärer Destillation (G1) durch eine Zweidruck-Methode oder einer zweiten Gruppe sekundärer Destillation (G2) durch eine Extraktions-Destillationsmethode zuzuordnen,
• eine fortgeschrittene Einheit, die zwei Extraktions-Hilfssäulen (2, 3) umfasst, die zur Durchführung einer Sekundärdestillation ausgelegt sind und um gleichzeitig eine Extraktionsdestillation mittels eines Treibmittels und eine Zweidruck-Destillation derart zu gestatten, dass alle chemischen Komponenten (C21, C22, C41, C42, C51, C52) jedes Untergemischs (N2, N4, N5) getrennt werden.

9. Trennsystem (S) nach Anspruch 8, wobei die fortgeschrittene Einheit aus zwei Hilfssäulen (2, 3) besteht, die dazu ausgelegt sind, die Durchführung entweder einer fortgeschrittenen Extraktionsdestillation oder einer fortgeschrittene Zweidruckdestillation zu gestatten.

10. Trennsystem (S) nach einem der Ansprüche 8 und 9, umfassend einen Lösungsmittel-Vorratsbehälter (7), der dazu ausgelegt ist, eine der zwei Hilfssäulen (2, 3) zu versorgen, wenn diese ein komplexes Untergemisch (N4, N5) der zweiten Gruppe (G2) erhält.

## Claims

1. Process for separating a plurality of chemical components (Cx) from a chemical mixture (M1-M4), the chemical mixture (M1-M4) comprising a plurality of fluorinated fluids (F1-F6), each fluorinated fluid (F1-F6) comprising at least one fluorinated chemical component (Cx), the process comprising:
- a step of identification (E2) in the chemical mixture (M1-M4) of at least two sub-mixtures (N1-N5), each sub-mixture (N1-N5) being a simple sub-mixture (N1, N3) comprising a single chemical component (C1, C4) or a complex sub-mixture (N2, N4, N5) comprising a combination of chemical components (C21, C22, C41, C42, C51, C52), each complex sub-mixture (N2, N4, N5) being associated either with a first secondary distillation group (G1) by a pressure swing distillation method, or with a second secondary distillation group (G2) by an extraction distillation method,
- a primary distillation step (E3), by means of a simple main distillation column (1), so as to separate each identified sub-mixture (N1-N5),
- a secondary distillation step (E4), by means of a same advanced unit of two auxiliary extraction columns (2, 3), of each complex sub-mixture (N2, N4, N5) of the first group (G1) and of the second group (G2), the complex sub-mixtures (N2) of the first group (G1) being separated by the pressure swing distillation method and the complex sub-mixtures (N4, N5) of the second group (G2) being separated, by means of a same solvent (Q), by the extraction distillation method, by the same two auxiliary columns (2, 3).

2. Separation process according to claim 1, wherein each complex sub-mixture (N2, N4, N5) is azeotropic or pinched.

3. Separation process according to one of claims 1 and 2, wherein the solvent (Q) is selected from the following solvents: n-pentane, dichloromethane, methyl isobutyl ketone (MIBK), methyl ethyl ketone (MEK), heptanone, pentanone, cyclohexanone and dimethylether.

4. Separation process according to one of claims 1 to 3, wherein at least one of the chemical components (Cx) of at least one sub-mixture (N1-N5) is a hydrofluoroolefin HFO, a hydrochlorofluoroolefin HCFO, a hydrofluorocarbon HFC or a hydrochlorofluorocarbon HCFC.

5. Separation process according to one of claims 1 to 4, wherein the identification step (E2) is performed from a database associating the sub-mixtures with a secondary pressure swing distillation method or a secondary extraction distillation method.

6. Separation process according to one of claims 1 to 5, comprising a preliminary step of collecting (E0) a plurality of fluorinated fluids (F1-F6) comprising different chemical components (Cx) and mixing several fluorinated fluids (F1-F6) from the fluorinated fluids (F1-F6) collected, so as to form at least one chemical mixture (M1-M4) of fluorinated fluids (F1-F6).

7. Method for recycling the chemical components (Cx) of a chemical mixture (M1-M4) of fluorinated fluids (F1-F6) comprising the steps (E1-E4) of the separation process according to one of claims 1 to 6 and a step of independent recycling of each chemical component (Cx).

8. Separation system (S) for implementing the method according to one of claims 1 to 6, the separation system (S) comprising:
- a simple main distillation column (1), configured to receive the chemical mixture (M1-M4) of fluorinated fluids (F1-F6) and implement a primary distillation, so as to separate the chemical mixture (M1-M4) into at least two sub-mixtures (N1-N5),
- an electronic calculator (6) configured to:
- identify in the chemical mixture (M1-M4) the different sub-mixtures (N1-N5), each sub-mixture (N1-N5) being a simple sub-mixture (N1, N3) comprising a single chemical component (C1, C3) or a complex sub-mixture (N2, N4, N5) comprising a combination of chemical components (C21, C22, C41, C42, C51, C52),
- associate each complex sub-mixture (N2, N4, N5) either with a first secondary distillation group (G1) by a pressure swing method, or with a second secondary distillation group (G2) by an extraction distillation method,
- an advanced unit comprising two auxiliary extraction columns (2, 3), configured to implement a secondary distillation and to allow both a distillation by extraction by means of an entrainer and a distillation by pressure swing, so as to separate all the chemical components (C21, C22, C41, C42, C51, C52) from each sub-mixture (N2, N4, N5).

9. Separation system (S) according to claim 8, wherein the advanced unit consists of two auxiliary columns (2, 3), configured to enable the implementation either of an advanced distillation by extraction or an advanced distillation by pressure swing.

10. Separation system (S) according to one of claims 8 and 9, comprising a solvent tank (7) configured to supply one of the two auxiliary columns (2, 3) when the latter receives a complex sub-mixture (N4, N5) of the second group (G2).
